(19) Europäisches Patentamt
European Patent Office
Office européen des brevets

(11) **EP 4 397 303 A1**

(12) **EUROPEAN PATENT APPLICATION**
published in accordance with Art. 153(4) EPC

(43) Date of publication:
**10.07.2024 Bulletin 2024/28**

(21) Application number: **22862454.0**

(22) Date of filing: **01.09.2022**

(51) International Patent Classification (IPC):
*A61K 31/03* (2006.01)     *A61K 31/045* (2006.01)
*A61K 31/047* (2006.01)     *A61K 31/05* (2006.01)
*A61K 8/67* (2006.01)

(52) Cooperative Patent Classification (CPC):
A61K 8/67; A61K 31/03; A61K 31/045;
A61K 31/047; A61K 31/05; A61K 36/185;
A61K 36/48; A61K 36/61; A61P 15/12; A61P 19/10

(86) International application number:
**PCT/BR2022/050350**

(87) International publication number:
**WO 2023/028683 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.09.2021 BR 102021017493**

(71) Applicants:
- **AGMONT, CARVALHO E BUSTAMANTE COMERCIO DE PRODUTOS NATURAIS S.A.**
  **04552-050, Vila Olímpia, São Paulo - SP (BR)**
- **Tavares Carvalho, José Carlos**
  **68902-689, Muca, Macapá - AP (BR)**

(72) Inventors:
- **LOPES DO NASCIMENTO, Aline**
  **68909-854 Macapá - AP (BR)**

- **RIBEIRO DA SILVA, Heitor**
  **68900-115 Macapá - AP (BR)**
- **ESPINOLA BUSTAMANTE, Osvaldo Alejandro**
  **01310-000 São Paulo - SP (BR)**
- **TAVARES DE LIMA TEIXEIRA DOS SANTOS, Abrahão**
  **Victor**
  **68902-280 Macapá - AP (BR)**
- **DA SILVA MONTE, Andreia Thais**
  **68903-383 Macapá - AP (BR)**
- **FLEXA ROCHA, Clarice**
  **68928-211 Santana - AP (BR)**
- **TAVARES CARVALHO, José Carlos**
  **68902-689, Muca, Macapá - AP (BR)**

(74) Representative: **EP&C**
**P.O. Box 3241**
**2280 GE Rijswijk (NL)**

(54) **NATURAL CLUSTER OR FORMULATION CONTAINING BIXA ORELLANA OIL WITH A STANDARDIZED TOCOTRIENOL CONTENT, DRY MYRCIARIA DUBIA EXTRACT WITH A STANDARDIZED ASCORBIC ACID CONTENT AND SOY GERM WITH A STANDARDIZED ISOFLAVONE CONTENT, METHOD FOR PREPARING SAME AND USE THEREOF FOR THE SUPPORT TREATMENT OF MENOPAUSE SYMPTOMS**

(57) The natural cluster or formulation containing oil from *Bixa orellana* standardized in tocotrienols, dry extract of *Myrciaria dubia* standardized in ascorbic acid and soy germ standardized in isoflavones for the supportive treatment for menopause and action on osteoporosis pertains to the technical sector of Pharmaceutical, Phytotherapeutic, Food Supplements and Nutraceuticals. The invention is developed according to techniques established in the scientific environment both for its preparation and for the quality control thereof, containing raw materials derived from nature. The cluster is in the form of granules and/or fine powder which is obtained after systematically mixing the components and industrial validation. The invention has an adequate release for what is expected for the form of the product and optimal flow properties. The process for the preparation of said natural cluster or formulation is also described.

**Description**

**FIELD OF THE INVENTION**

This invention relates to a formulation comprising a combination of three plant-based actives (oil from *Bixa orellana* (OBo) standardized in tocotrienols, dry extract of *Myrciaria dubia* standardized in ascorbic acid (Mdaa), and soy germ standardized in isoflavones (GSIf)) to obtain a natural cluster for the supportive treatment of menopause symptoms and action on osteoporosis. The process for the preparation of said formulation or natural cluster is also described.

**BACKGROUND OF THE INVENTION**

A search in national and international patent databases did not find any record of a similar product or one that could compete with this natural cluster and/or its other pharmaceutical forms.

This invention can be applied to the technological development of various pharmaceutical form such as capsules, powders, tablets, granulates, among others, involving the use of oil from *B. Orellana* standardized in tocotrienols, the extract of *M. dubia* standardized in ascorbic acid, and soy germ standardized in isoflavones.

The natural cluster, the object of this patent, is a granulate composed of the lipophilic actives of the oil from *Bixa orellana* standardized in tocotrienols, and the polar actives from soy germ standardized in isoflavones and from the extract of *M. dubia.*

Soy has increasingly garnered attention from food, pharmaceutical, and cosmetic industries due to its nutritious components, nutraceutical activities, and active metabolites such as saponins, triterpenes, phenolic acids, flavonoids, proteins, fatty acids, and isoflavones.

Soy isoflavones, being considered the main beneficial components of the grain, have triggered considerable interest in research, with a daily intake of 20 to 100 mg/day established to achieve the desired effect.

They also show preventive effects for osteoporosis, cardiovascular diseases, some cancers (colon, breast, and prostate), acting by reducing menopause symptoms as well as anxiety and depression.

The prevalence of depression in women is higher than in men, and this data is related to reproductive hormones estradiol and progesterone in the etiology of the disease. Isoflavone aglycones are the isoflavones that present the greatest pharmacological activity as an adjunct for the treatment of menopause symptoms.

Regular consumption of isoflavones allows better digestibility and biotransformation by the intestinal microbiota. Isoflavones are part of the Asian diet with greater consumption in quantity compared to Western diets.

A cross-sectional study was conducted by Yu et al. (2015) in a rural community in northeastern China with 1,717 residents aged 65 and older, a population with regular soy consumption averaging 4 times a week, thus having a lower chance of depressive states compared to those with average consumption of 2 to 3 times a week. In Hong Kong, Woo et al. (2006) conducted a cross-sectional study with 3,999 elderly and observed that isoflavone intake was inversely related to the Geriatric Depression Scale score.

Camu-camu [*Myrciaria dubia* (H.B.K.) McVaugh Myrtaceae] is a typically Amazonian fruit found naturally along the edges of rivers, lakes, and flooded forests both in dark and clear waters. It has attracted interest due to its concentration in ascorbic acid, showing a variation of 845 to 3,133 mg per 100 g of whole pulp.

Zhu et al. (2012) observed for the first time that ascorbic acid was able to prevent bone density loss in mice that had their ovaries removed (a procedure known to trigger the problem), suggesting that the nutrient may help prevent osteoporosis. The joint action of ascorbic acid with polyphenols presents in the *M. dubia* extract (approximately 1797.0 mg equivalent to gallic acid - EAG.100g$^{-1}$) provides antioxidant and beneficial effects against menopause symptoms.

**OBJECTIVES OF THE INVENTION**

A primary objective of this invention is to provide a formulation or natural cluster useful in the supportive treatment of menopause symptoms and action on osteoporosis.

Another objective is to describe a process or method to produce said formulation or natural cluster.

**SUMMARY OF THE INVENTION**

The first objective of this invention is achieved through a formulation or natural cluster comprising 35 - 45% of oil from *Bixa orellana* standardized in tocotrienols, 1 - 5% of lecithin, 1 - 5% of dry extract of *Myrciaria dubia* standardized in ascorbic acid, and 15 - 25% of soy germ standardized in isoflavones, in addition to 20 - 48% of excipients.

This formulation or natural cluster being used in the supportive treatment of menopause symptoms and action on osteoporosis.

The second objective is achieved by a 3oder3so f method for producing said formulation or natural cluster, comprising the steps of:

a) dispersion and homogenization of the lecithin in the standardized oil of *Bixa orellana*, where the amount of standardized oil should be weighed first and then the lecithin added; and
b) incorporation of the solid components into the oil/lecithin mixture

where the components in the powder form are weighed separately and then mixed and homogenized intensely so that only after this procedure they can be mixed with the oil/lecithin to obtain a compact mass where the process of preparing the mass is carried out through the incorporation of small quantities of the powder mixture onto the oil/lecithin mixture under slow careful mixing.

## DESCRIPTION OF THE FIGURES

In figures 1a, 1b, 1c, 1d, 1e, and 1f, the chromatographic profile of samples of the active (tocotrienols) obtained by high-efficiency liquid chromatography (UHPLC) coupled to the UV detector at 220 nm (blue), 254 nm (pink), 300 nm (black), and 340 nm (maroon) corresponding to 6 batches of the same active can be observed.

In figure 2, the physical-mechanic properties of the formulation can be observed.

In figure 3, the infrared spectrum region of the formulation's excipients, of the oil from *Bixa orellana* standardized in tocotrienols, and the formulation are observed.

In figure 4, photomicrography of the formulation is observed.

In figure 5, the dissolution profile of the final obtained formulation expressed in total tocotrienol content released is observed.

In figure 6, the biological activities as well as the mechanism of action of each molecule analyzed from soy germ (isoflavones) in the CDDI server can be observed.

In figure 7, the docking between the complex and the isoflavones (daidzein, genistein, and glycitein) is observed.

## DETAILED DESCRIPTION OF THE INVENTION

For definition purposes, unless explicitly expressed otherwise, all percentages mentioned herein are based on the weight by weight (w/w) ratio.

In one embodiment, a formulation or natural cluster containing 35 - 45% of oil standardized in tocotrienols from *Bixa orellana,* 1 - 5% of lecithin, 1 - 5% of dry extract of *Myrciaria dubia* standardized in ascorbic acid, and 15 - 25% of soy germ standardized in isoflavones, and 20 - 48% of excipients by weight relative to the total weight of the formulation or natural cluster is described.

In a preferred embodiment, the oil from *Bixa orellana* standardized in tocotrienols is obtained by an alkaline lixiviation process from the seeds. A technician in the field would have routine knowledge of the alkaline leaching process, which is already traditional in the state of the art.

In a preferred embodiment, the dry extract of *Myrciaria dubia* is obtained by standard drying in a Spray Dryer. Just as explained above, the technician in the field is familiar with the Spray Dryer technique, which is already known by the state of the art.

In a preferred embodiment, soy germ obtained by pressing. Similarly, as explained above, the technician in the field is familiar with the pressing technique, which is already known by the state of the art.

In a preferred embodiment, the excipients are corn starch, maltodextrin, microcrystalline cellulose, and silicon dioxide.

In a preferred embodiment, the lecithin is sunflower lecithin.

In a preferred embodiment, the oil standardized in tocotrienols from *Bixa orellana* has a reference chemical constitution of 1 - 5% Bixin, 5 - 15% tocotrienols as compounds of interest.

In a preferred embodiment, the dry extract from *Myrciaria dubia* is standardized in ascorbic acid between 1 and 4.5%.

In a preferred embodiment, the soy germ is standardized in isoflavones between 4 and 8%.

In a preferred embodiment, the formulation or cluster herein described has a pH within the range of 5.0 to 9.0. In a preferred embodiment, said pH is within the range considered "neutral". Although the term "neutral" is self-explanatory to a person skilled in the art, for the sake of better definition, a pH within the neutrality range can be understood as any pH close to 7.0, for example, but without limiting the present invention to a pH within the range of 5.5 to 8.5 or 6.0 to 8.0.

Said formulation or natural cluster, in a preferred embodiment, is presented in a final form of granulate and/or fine powder.

Furthermore, preferably, the formulation or natural cluster can be presented in the form of capsules, tablets, powder, or any other pharmaceutical form of controlled release or not.

Naturally, a person skilled in the art possesses in their repertoire of knowledge, because of routine work, the understanding that adaptations may be necessary in terms of additional excipients in order to make the formulation or natural cluster available in the different forms described above. In any case, said adjustments are based on knowledge available and commonplace for a person skilled in the art, so they do not require further detailing in the present invention.

The formulation described herein has demonstrated efficiency in the supportive treatment of menopause symptoms and action on osteoporosis.

As is known by a person skilled in the art, menopause is the transition period in which a woman moves from the reproductive phase to the non-reproductive phase, also called post menopause, marked by a progressive decrease in the amount of hormones produced. The typical symptoms of this transition phase include, for example: sudden hot flashes; decreased sexual appetite; dizziness and palpitations; insomnia and poor quality of sleep; night sweats; itching and vaginal dryness; discomfort during sexual intercourse; loss of skin elasticity; decreased breast size; depression and irritability; weight gain; headache and lack of concentration; urinary incontinence when exerting effort; and joint pain.

Therefore, the use or application of said formulation or natural cluster for the preparation of a medicine or pharmaceutical composition, phytotherapeutic composition, a dietary supplement, or a nutraceutical for the supportive treatment of menopause symptoms and osteoporosis is also described herein.

In a new realization, the method or process for the production/acquisition of the formulation or natural cluster containing OBo, Mdaa, and GSIf is also described.

In a preferred embodiment, said method or process comprises the steps:

a) dispersion and homogenization of the lecithin in the standardized oil of *Bixa orellana*, where the amount of standardized oil should be weighed first and then the lecithin added; and

b) incorporation of the solid components into the oil/lecithin mixture

where the components in powder form are weighed separately and then mixed and homogenized intensely so that only after this procedure they can be mixed with the oil/lecithin to obtain a compact mass where the process of preparing the mass is carried out through the incorporation of small quantities of the powder mixture into the mixing bowl of a planetary mixer containing the oil/lecithin mixture, and the mixing process is started at low speed in the mixer. The addition of powder in small quantities and under constant stirring is maintained until the total incorporation of the powders into the oil/lecithin.

In a preferred embodiment, the first step involves dispersion and homogenization of sunflower lecithin in OBo. The mixture is made in a planetary mixer, where the amount of oil (35 to 45%) should be weighed first and then the lecithin in the required amount (1 to 5%) is added.

After the first step, the composition of OBo in the formulation or natural cluster of this invention becomes differentiated because, in addition to having a certain amount of tocotrienols in its composition coming from the oil standardized in tocotrienols from *Bixa orellana,* it now has a greater quantity of phospholipids that facilitate the formation of particles that are adsorbed onto the material used for the production of the final granulate.

The additional phospholipids used in the invention come from lecithin, preferably from sunflower lecithin, widely used for obtaining food and pharmaceutical products and considered a natural surfactant. Technically, it can be obtained from egg yolk and various other sources of vegetable oils, with soy being the most common source (with percentages of 2% to 3% lecithin) due to its greater availability and emulsifying properties.

Lecithins, in general, are formed by a mixture of phospholipids (50%), triglycerides (35%), and glycolipids (10%), carbohydrates, carotenoid pigments, and other micro compounds. The surfactant properties of lecithins come from the molecular structure of phospholipids, their active components. These are formed by a hydrophobic portion and a hydrophilic portion, with properties like the oil standardized in tocotrienols of the invention.

In a preferred embodiment, the lecithin used in the first step of the present method or process is sunflower lecithin.

The second step involves the incorporation of the solid components into the oil/lecithin mixture. These solid components are the soy germ standardized in isoflavones and the dry extract of *M. dubia* standardized in ascorbic acid, as already discussed above, and excipients.

In a preferred embodiment, the excipients are corn starch, maltodextrin, microcrystalline cellulose, and silicon dioxide.

All components used in the present method or process are incorporated in their final quantities as discussed above.

The ingredients, typically in powder form, are weighed separately and placed in appropriately sized containers, for example, plastic bags. At the end of weighing, they are intensely mixed and homogenized altogether so that after this procedure, they are added to the oil/lecithin mixture to obtain a compact mass.

The process of preparing the mass is carried out through the incorporation of small quantities of the powder mixture into the bowl of the planetary mixer containing the oil/lecithin mixture, with the mixing starting in the mixer at a low speed. The addition of powder in small quantities and under continuous stirring is maintained until the total incorporation of the powders into the oil/lecithin.

The result of these operations culminates in obtaining a formulation or natural cluster in the form of granulate and/or fine powder containing the three active plant-based principles. It is worth highlighting the novelty of the combination of the three actives as proposed in the invention.

In an alternative embodiment, the granulate can still be processed by known methods so that the final presentation of this formulation or natural cluster is in the form of a fine powder.

The use of products derived from plants without a specific quality control is a serious problem faced by the general community. There is a need for meticulous quality control regarding the concentrations of the chemical markers of interest for the standardization of the extracts and oils of OBo, Mdaa, and GSIf used as described above, so that the credibility of phytoproducts is reinforced compared to synthetics, which consequently translates as new treatment and prevention options available to the population and the market.

As part of the pre-formulation study, the active ingredients in question were studied according to the chemical markers: tocotrienols for *B. orellana* oil, ascorbic acid for the dry extract of *M. dubia,* and isoflavones for the soy germ.

The analyses of tocotrienols and isoflavones were performed using high-efficiency liquid chromatography equipment (UHPLC UltiMate 3000, Thermo Fisher Scientific) coupled to an ultraviolet (UV) detector. A C-18 column (250 mm × 4.6 mm × 5 $\mu$m) at 30 $\pm$ 1°C was used as the stationary phase, and a mixture of methanol and ultrapure water (95:5 v/v) with a flow rate of 1.2 ml/min as the mobile phase. The separation of the constituents of the samples was monitored at wavelengths of 220, 254, 300, and 340 nm. For the determination of total tocotrienols, the results obtained at 300 nm were used, and for isoflavones, those obtained at 254 nm.

Ascorbic acid (vitamin C) was determined by Titrimetry with 2,6-dichlorophenolindophenol (DFI) at 0.5% with oxalic acid (AOAC 1984).

The physical-mechanic properties of the formulation were evaluated, and all analyses were performed in triplicate.

To determine possible chemical interactions between the actives and excipients, FT-IR spectra were obtained using an IRAffinity equipment (Shimadzu, Japan). The spectra were recorded in transmission mode between 4000 and 400 cm$^{-1}$ with a resolution of 0.44 cm$^{-1}$. Each sample was compressed with KBr (1:100 w/w) before acquisition, and the background value of pure KBr was acquired before the samples were analyzed.

The particle size distribution was carried out on samples using sieves with mesh sizes of 740 $\mu$m, 590 $\mu$m, 350 $\mu$m, 250 $\mu$m, 177 $\mu$m, 125 $\mu$m, and 74 $\mu$m (IC London, UK). The sieves were shaken for 10 minutes.

Quantities of the formulation were placed on Petri dishes and stored in a desiccator at 25°C for 1 week. The moisture absorption isotherms were determined by gravimetry. In this case, 1.0 g of the formulation was placed in closed chambers with saturated solutions of different inorganic salts.

Thus, distilled water (100% RH), potassium chloride (84%), sodium chloride (75%), potassium thiocyanate (47%), and calcium chloride (31%), and activated silica gel at 105 °C for three hours for RH=0. The sample was weighed at intervals of 12 hours until equilibrium moisture content was reached. The equilibrium moisture absorption (EMS) was determined using the expression:

$$EMS = (Mi / M) \times 100$$

Where Mi is the moisture absorbed at equilibrium and M is the weight of dry powder. The moisture absorption profile was obtained, and for this, a graph of residual moisture percentage versus relative humidity was made. The graph was adjusted using the least squares method expressing the relationship as a curve y=mX + a.

Micrographs of the formulation surface were obtained through scanning electron microscopy (SEM) using a T3030 Plus Tabletop microscope (Hitachi, Japan) operated at 15 kV.

The dissolution profile was based on the method described by the Brazilian Pharmacopeia. It was conducted on a dissolution equipment with six vessels model 299 (Nova Etica Ltda., São Paulo, Brazil).

The simulated gastric juice solution used as the immersion liquid consisted of 16% (mass/volume) pepsin dissolved in 0.1 mmol/L HCl (57633 FIP-U/100g). The pancreatin-bile mixture was prepared by dissolving 0.4% (mass/volume) pancreatin and 0.76% (mass/volume) bile in 0.1 mmol/L sodium bicarbonate (NaHCO3).

In each 900 mL vessel, the simulated gastric juice solution was the dissolution medium, the agitation speed was 75 rpm.

Dissolution was established by quantifying total tocotrienols, isoflavones, and ascorbic acid released into the digestive juice at times of 5, 10, 15, 20, 25, 30, 35, 40, 45, 50, 55, and 60 minutes, taking 5 mL aliquots filtered through filter paper (12.5 cm diameter). The verification of the content of total tocotrienols and isoflavones present in the formula was determined by the method validated by gas chromatography and UHPLC. Ascorbic acid (vitamin C): determined by titrimetry with 2,6-dichlorophenolindophenol (DFI) at 0.5% with oxalic acid (AOAC 1984).

The chemical structure of molecular active markers for the pharmacological activities studied was first confirmed on the *PubChem* website (https://pubchem.ncbi.nlm.nih.gov/). Then, the structures of tocotrienols and geranylgeraniol were obtained using *ChemDraw* software (COUSINS 2011) and optimized in *HyperChem* software by the semi-empirical RM1 method (NAGAMANI et al. 2018).

For the prediction of activities of molecular active markers, the *PASS* server (Prediction of Activity Spectra for Substances) was used. For the study of possible biological targets, the SEA server (similarity ensemble approach) was employed, which has affinity with the submitted structures according to a chemical similarity approach (WANG et al. 2016).

Cortellis Drug Discovery Intelligence (CDDI) was used to verify existing studies about the mechanisms of action of molecular active markers and the targets used. The analysis included pre-clinical and clinical studies, thus providing the study with speed, safety, and reliability (SHIH et al. 2018).

The selected targets were submitted to the GOLD 2020.1 program to investigate the interaction modes and docking between the molecular active markers and the biological targets that possess dyslipidemic and anti-inflammatory activities. All targets submitted for docking were first validated using the Root-Mean-Square Deviation (RMSD) values as a parameter.

For the ADME prediction (absorption, distribution, metabolism, and excretion), the *PreADMET* web server (https://preadmet.bmdrc.kr/) and the *SwissADME* web server (http://www.swissadme.ch) were used. These servers calculated the properties of human intestinal absorption (HIA) and permeability in cells derived from human colon adenocarcinoma (Caco-2) and canine kidney cells Madin-Darby (MDCK). Moreover, they determined the distribution and metabolism properties such as the percentage of binding to plasma proteins (%PPB), penetration of the blood-brain barrier (BBB), permeability of the glycoprotein (P-gp), and possible inhibitions of the cytochromes P450 known as Cyp (DAINA et al. 2017; NUNES et al. 2020).

The prediction of the toxicological properties of the molecules was performed on two web servers. The toxicity prediction by the *PreADMET* web server was based on the Ames Test (this method was used to test the mutagenicity of compounds in strains of the bacterium *Salmonella typhimurium*) and Carcinogenicity in rodents based on data from the NTP (National Toxicology Program) and according to the FDA, resulting from in vivo Carcinogenicity in mice and rats over 2 years (RICHA et al. 2017; ZEIGER 2019). The *ProTox*-II platform conducted the toxicity prediction of molecules in various classifications: acute toxicity, organ toxicity through hepatotoxicity, and this server determines at the cellular level carcinogenicity, immunotoxicity, mutagenicity, and cytotoxicity (BANERJEE et al. 2018).

The product obtained in this invention shows active characteristics in *in silico* prediction both for pharmacokinetic-receptor activities with respect to its pharmacological activity and predicted low toxicity.

Molecular docking was performed between hydroxysteroid dehydrogenase (17 HSD1) complexed with 17-beta-estradiol and the isoflavones present in the invention. This enzyme was found on the PDB site and presents the ID code: 1IOL. It plays an important role in the biotransformation or formation of estrogens that are active in gonadal and peripheral tissues. The 17-HSD1 converts estrone into 17β-estradiol, which is a more active estrogen.

After validating the docking with this target, the docking process was performed for each molecule (daidzein, genistein, and glycitein) and also the complexed molecule to compare the values of the Gold score and the interactions between the active site.

In the analysis, it was possible to observe that all the molecules submitted to molecular docking presented various interactions with the biological target, with at least one of these with amino acids of interest for the biological response. Among these interactions, possible to be visualized in the best models of poses, electrostatic interactions, hydrogen bonding, and hydrophobic Pi and aromatic interactions were observed.

In the docking, it was observed that the Gold score values of the isoflavone molecules daidzein, genistein, and glycitein (score: 52.78, 54.33, 50.31, respectively) were not superior to the score value (60.05) of the complexed molecule (17-beta-estradiol). The 17-beta-estradiol molecule interacted with the amino acids Ser142 and Tyr155, which are from the activity site mentioned above, in comparison, the complexed molecule daidzein interacted with Glu282, genistein interacted with Ser142 and Glu282, and finally, glycitein interacted with Tyr155. The above results prove a benefit in the supportive treatment of menopause symptoms.

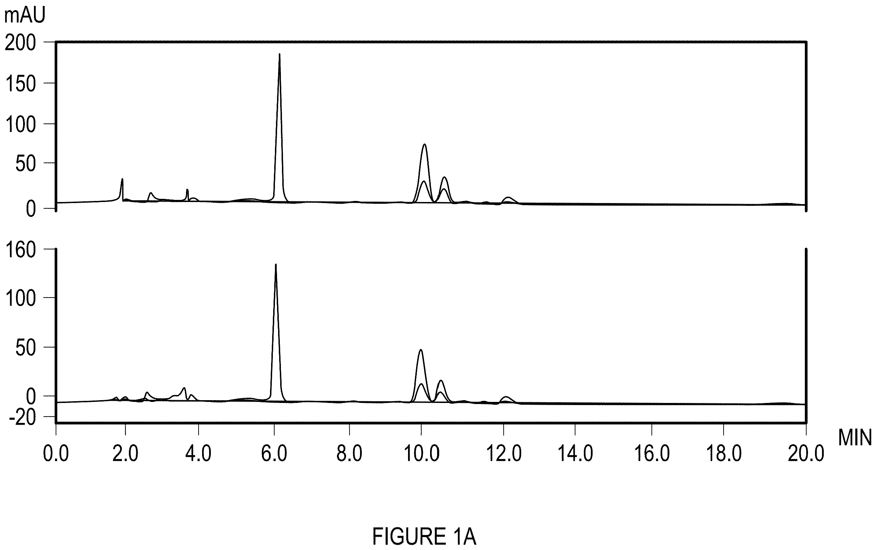

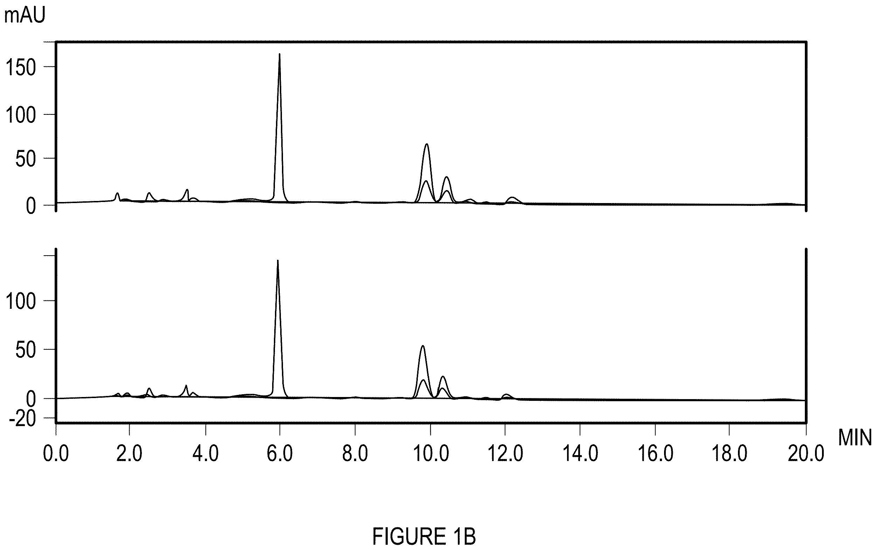

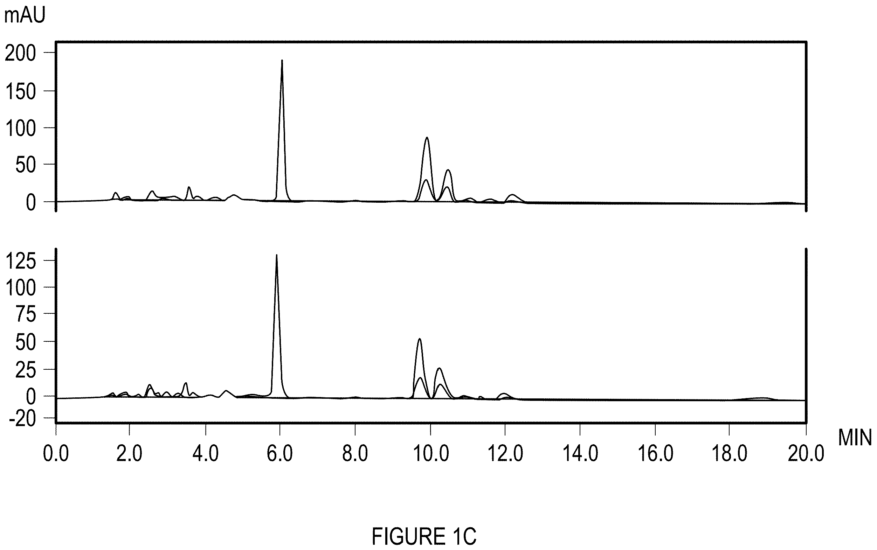

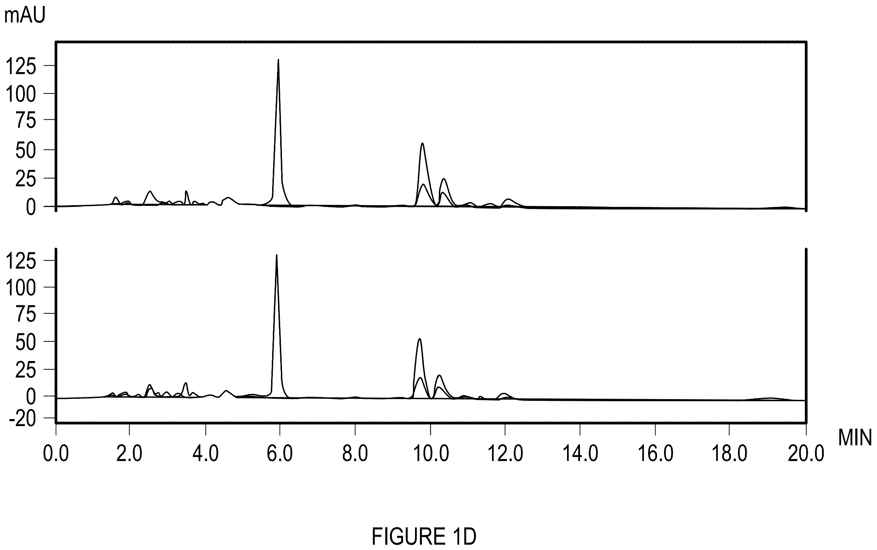

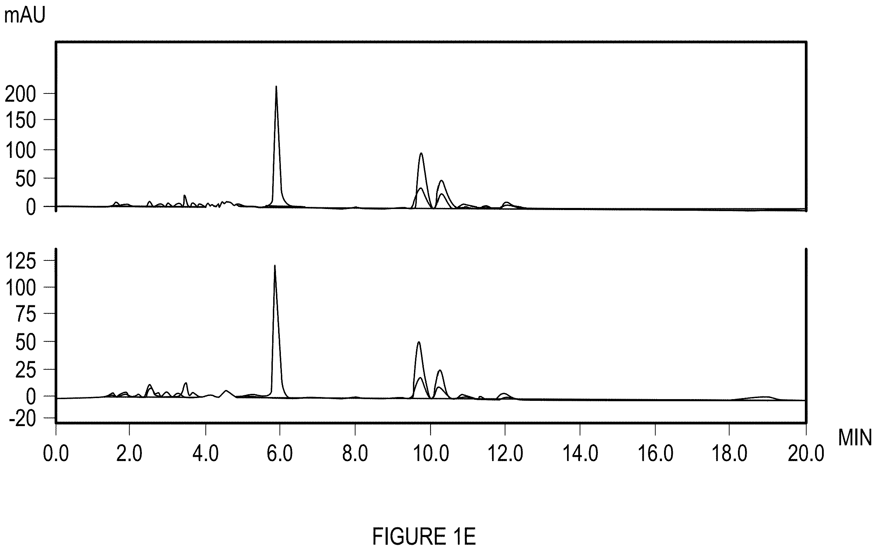

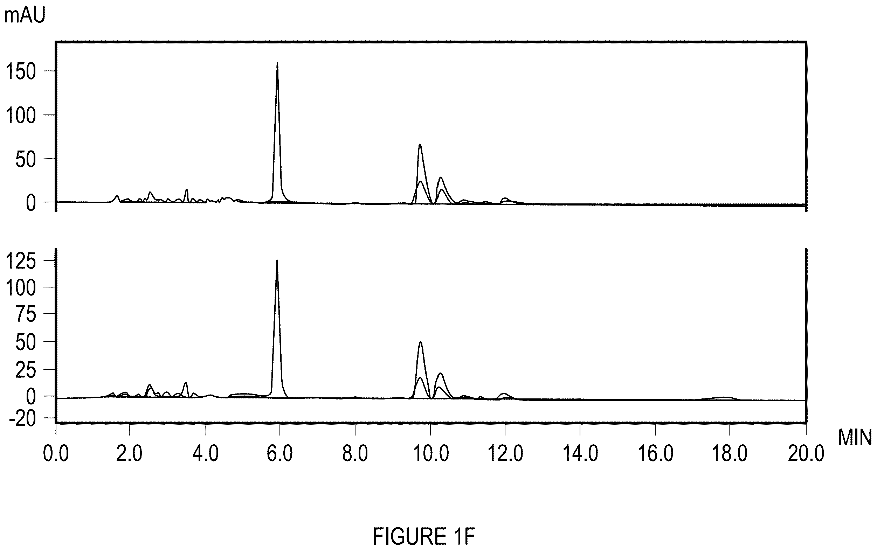

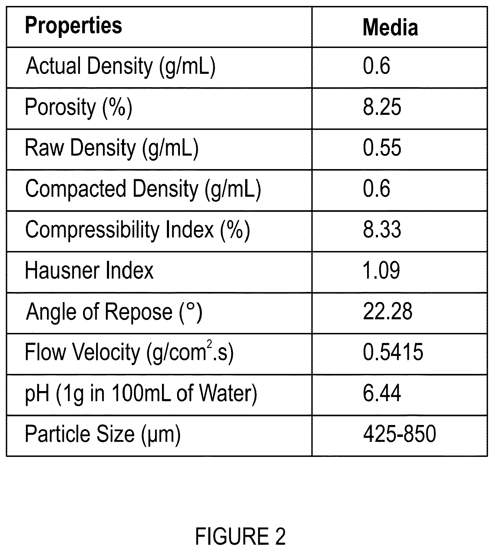

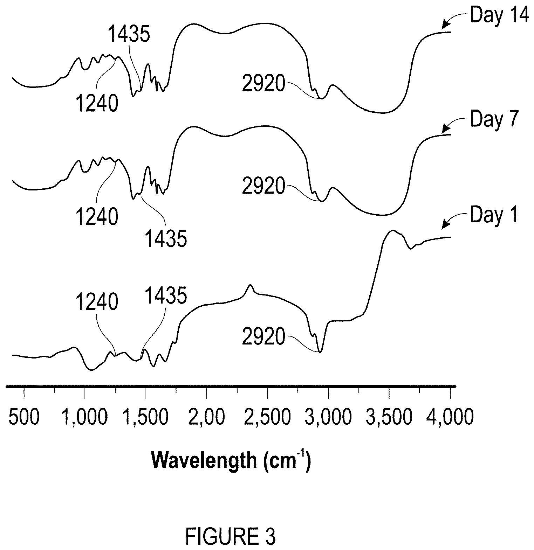

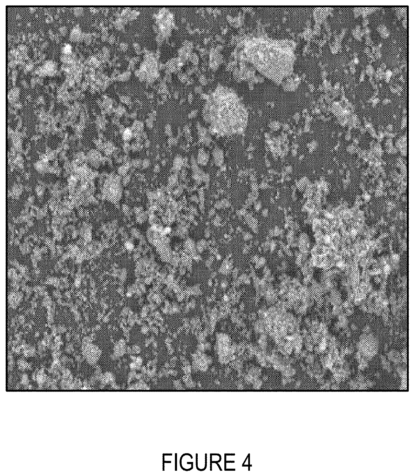

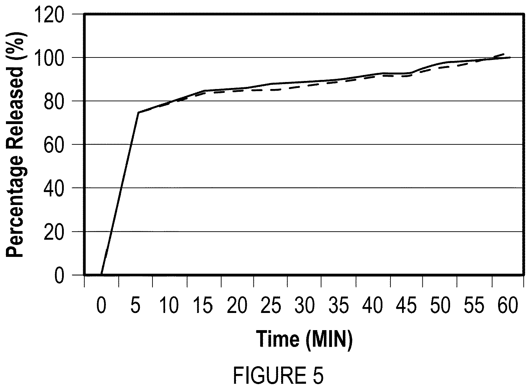

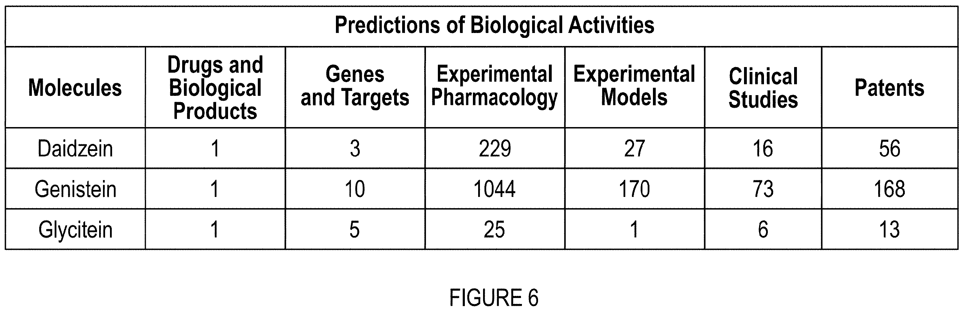

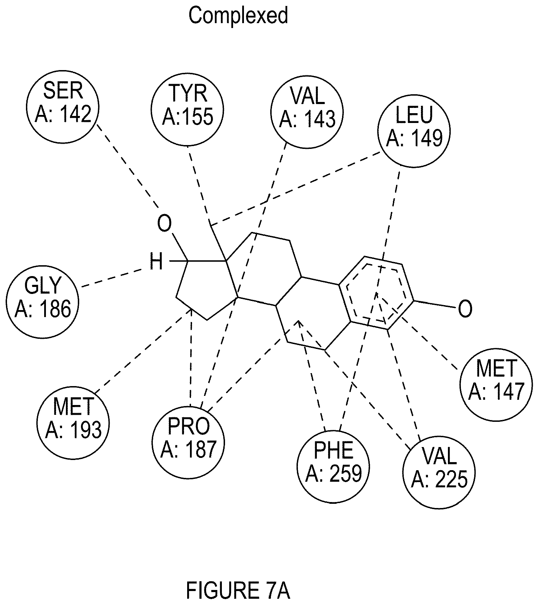

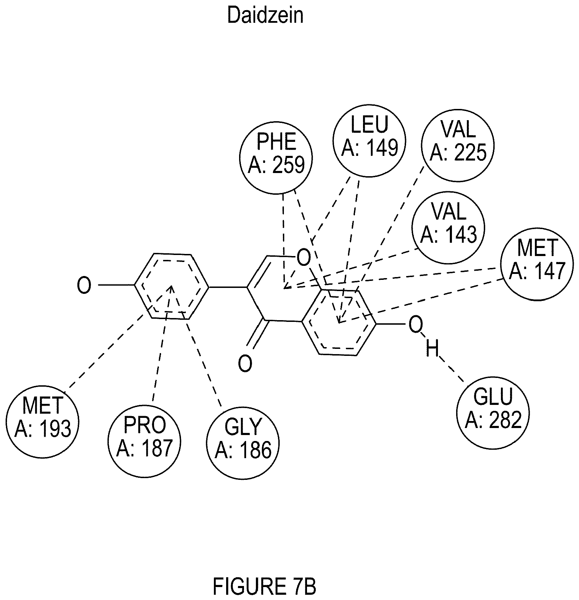

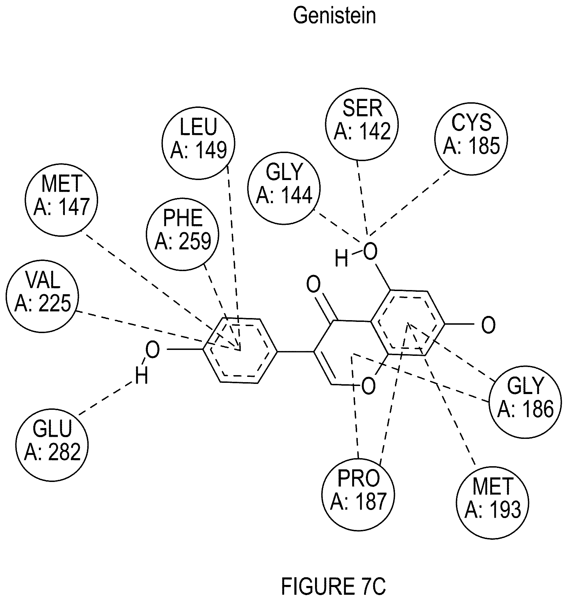

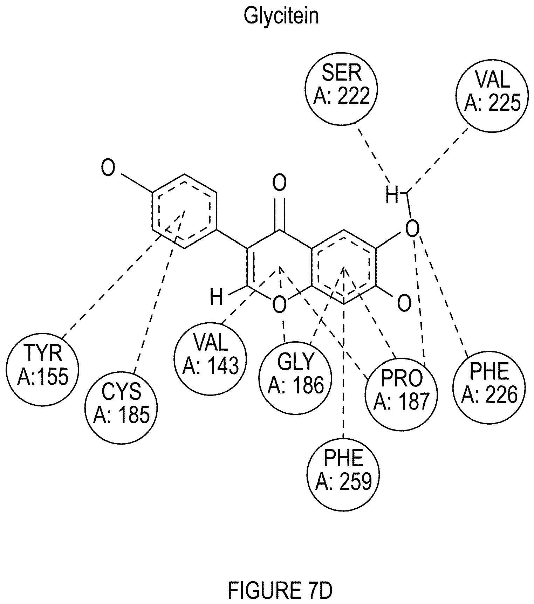

The natural cluster containing oil from *Bixa orellana* standardized in tocotrienols dry extract from *Myrciaria dubia* standardized in ascorbic acid, and soy germ standardized in isoflavones according to the invention is additionally explained through figures 1 to 7.

## REFERENCES

Abdul-Majeed S., N. Mohamed, I.N. Soelaiman, Effects of tocotrienol and lovastatin combination on osteoblast and osteoclast activity in estrogen-deficient osteoporosis, Evid Based Complement Alternat Med 2012 (2012) 960742.

Bhasin S, Calof OM, Storer TW, Lee ML, Mazer NA, Jasuja R, et al. Drug insight: Testosterone and selective androgen receptor modulators as anabolic therapies for chronic illness and aging. Nat Clin Pract Endocrinol Metab. 2006; 2(3):146-59.

Bonfim MR, Oliveira ASB, Amaral SL, Monteiro HL: Tratamento das Dislipidemias com Estatinas e Exercícios Físicos: Evidências Recentes das Respostas Musculares. Arq Bras Cardiol 2015; 104(4):324-332.

Carvalho, HO; Farias e Souza, BS; Santos, IVF; Resque, RL; Keita, H; Fernandes, CP; Carvalho, JCT: Hypoglycemic effect of formulation containing hydroethanolic extract of Calophyllum brasiliense in diabetic rats induced by streptozotocin. Rev bras farmacogn 2016.

Chin K.Y. S. Ima-Nirwana, Effects of annatto-derived tocotrienol supplementation on osteoporosis induced by testosterone deficiency in rats, Clin Interv Aging 9 (2014) 1247-1259.

Deng, L., Ding, Y., Peng, Y., Wu, Y., Fan, J., Li, W., ... Fu, Q. (2014). γ-Tocotrienol protects against ovariectomy-induced bone loss via mevalonate pathway as HMG-CoA reductase inhibitor. Bone, 67, 200-207, doi:10.1016/j.bone.2014.07.006 .

Doblaré, M.; Garci, J. M.; Goméz, M. J. Modelling bonne tissue fracture and heling a review. Engineering Fracture Mechanics, Oxford, v. 71, p. 1809-1840, 2004.

Idris A.I. (2012) Ovariectomy/Orchidectomy in Rodents. In: Helfrich M., Ralston S. (eds) Bone Research Protocols. Methods in Molecular Biology (Methods and Protocols), vol 816. Humana Press, Totowa, NJ.

Manolagas SC, Kousteni S, Jilka RL. Sex steroids and bone. Recent Prog Horm Res.

2002;57:385-409.

Palma, M. N. N., Rocha, G. C., Valadares Filho, S. C., Detmann E. Evaluation of Acid Digestion Procedures to Estimate Mineral Contents in Materials from Animal Trials. Asian Australas. J. Anim. Sci. Vol. 28, No. 11: 1624-1628. 2015.

Pearce BC, Parker RA, Deason ME, Qureshi AA, Wright JJ. Hypocholesterolemic activity of synthetic and natural tocotrienols. J Med Chem. 1992;35:3595-606.

Qureshi AA, Khan DA, Saleem S, Silswal N, Trias AM, Tan B, et al. Pharmacokinetics and Bioavailability of Annatto δ-tocotrienol in Healthy Fed Subjects. 2015;6.

Riggs BL, Khosla S, Melton LJ 3rd. A unitary model for involutional osteoporosis: estrogen deficiency causes both type I and type II osteoporosis in postmenopausal women and contributes to bone loss in aging men. J Bone Miner Res. 1998;13(5):763-73.

Sakamoto K, Kimura J. Mechanism of statin-induced rhabdomyolysis. J Pharmacol Sci. 2013;123:289-94.

Souza MO, Silva M, Silva ME, Oliveira RP, Pedrosa ML: Diet supplementation with acai (Euterpe oleracea Mart.) pulp improves biomarkers of oxidative stress and the serum lipid profile in rats. Nutrition. 2010; 26: 804-810.

Tan B, Watson RR, Preedy VR. Tocotrienols: Vitamin E Beyond Tocopherols. 2nd ed; 2013.

Vanderschueren D, Laurent MR, Claessens F, Gielen E, Lagerquist MK, Vandenput L, et al. Sex steroid actions in male bone. Endocr Rev. 2014;35(6):906-60.

Wong, S. K., Chin, K.-Y., Suhaimi, F. H., Ahmad, F., & Ima-Nirwana, S. (2018). Exploring the potential of tocotrienol from Bixa orellana as a single agent targeting metabolic syndrome and bone loss. Bone, 116, 8 21. doi:10.1016/j.bone.2018.07.003

**Claims**

1. A natural formulation or cluster **characterized by** comprising 35 - 45% of oil from *Bixa orellana* standardized in tocotrienols, 1 - 5% of lecithin, 1 - 5% of dry extract of *Myrciaria dubia* standardized in ascorbic acid, and 15 - 25% of soy germ standardized in isoflavones, and 20 - 48% of excipients by weight relative to the total weight of the natural formulation or cluster.

2. A natural formulation or cluster according to claim 1 **characterized by** the fact that the oil from *Bixa orellana* standardized in tocotrienols is obtained by the process of alkaline lixiviation from the seeds and the dry extract of *Myrciaria dubia* is obtained by standard drying in Spray Dryer and the soy germ is obtained by pressing.

3. A natural formulation or cluster according to claim 1 or 2 **characterized by** the fact that the lecithin is sunflower lecithin.

4. A natural formulation or cluster according to any of the claims 1 to 3 **characterized by** the fact that the excipients are corn starch, maltodextrin, microcrystalline cellulose, and silicon dioxide.

5. A natural formulation or cluster according to any of the claims 1 to 4 **characterized by** having oil from *Bixa orellana* standardized in tocotrienols with a reference chemical composition of 1 - 5% Bixin, 5 - 15% tocotrienols as compounds of interest, dry extract of *Myrciaria dubia* standardized in ascorbic acid between 1 and 4.5%, and the soy germ standardized in isoflavones between 4 and 8%, where the above values are expressed in w/w.

6. A natural formulation or cluster according to any of the claims 1 to 5 **characterized by** having a pH between 5.0 and 9.0.

7. A natural formulation or cluster according to any of the claims 1 to 6 **characterized by** the fact that it is used in the supportive treatment of menopause symptoms and action on osteoporosis.

8. A natural formulation or cluster according to any of the claims 1 to 7 **characterized by** having a final form of a granulate and/or fine powder.

9. A natural formulation or cluster according to any of the claims 1 to 8 **characterized by** the fact that it can be in the form of capsules, tablets, powder, or any other pharmaceutical form of controlled release or not.

10. The method for producing the natural formulation or cluster as defined in any of the claims 1 to 9 is **characterized by** comprising the following steps:

   a) dispersion and homogenization of the lecithin in the standardized oil of *Bixa orellana,* where the amount of standardized oil should be weighed first, and then the lecithin is added; and
   b) incorporation of the solid components into the oil/lecithin mixture

where the ingredients in the powder form are weighed separately and then mixed altogether and homogenized intensely so that only after this procedure they are mixed with the oil/lecithin to obtain a compact mass and the process of preparing the mass is carried out through the incorporation of small quantities of the powder mixture onto the oil/lecithin mixture followed by slow mixing.

11. The method according to claim 10 **characterized by** the fact that the lecithin is sunflower lecithin.

12. The method according to claim 10 or 11 **characterized by** the fact that the mixture of steps a) and b) is performed in a planetary mixer.

13. The method according to any of the claims 10 to 12 **characterized by** the fact that the powder components consist of soy germ standardized in isoflavones, dry extract of *M. dubia* standardized in ascorbic acid accordingly, and excipients.

14. The method according to any of the claims 10 to 13 **characterized by** the fact that the excipients are corn starch, maltodextrin, microcrystalline cellulose, and silicon dioxide.

15. The use of the natural formulation or cluster as defined in any of the claims 1 to 9 **characterized by** the fact of being used in the preparation of a medicine or pharmaceutical composition, phytotherapeutic composition, a dietary supplement, or a nutraceutical for the supportive treatment of menopause symptoms and action on osteoporosis.

FIGURE 1A

FIGURE 1B

EP 4 397 303 A1

FIGURE 1C

FIGURE 1D

FIGURE 1E

EP 4 397 303 A1

FIGURE 1F

EP 4 397 303 A1

| Properties | Media |
|---|---|
| Actual Density (g/mL) | 0.6 |
| Porosity (%) | 8.25 |
| Raw Density (g/mL) | 0.55 |
| Compacted Density (g/mL) | 0.6 |
| Compressibility Index (%) | 8.33 |
| Hausner Index | 1.09 |
| Angle of Repose (°) | 22.28 |
| Flow Velocity (g/com$^2$.s) | 0.5415 |
| pH (1g in 100mL of Water) | 6.44 |
| Particle Size (μm) | 425-850 |

FIGURE 2

FIGURE 3

FIGURE 4

FIGURE 5

| Predictions of Biological Activities | | | | | | |
|---|---|---|---|---|---|---|
| Molecules | Drugs and Biological Products | Genes and Targets | Experimental Pharmacology | Experimental Models | Clinical Studies | Patents |
| Daidzein | 1 | 3 | 229 | 27 | 16 | 56 |
| Genistein | 1 | 10 | 1044 | 170 | 73 | 168 |
| Glycitein | 1 | 5 | 25 | 1 | 6 | 13 |

FIGURE 6

Complexed

FIGURE 7A

Daidzein

FIGURE 7B

Genistein

FIGURE 7C

Glycitein

FIGURE 7D

## INTERNATIONAL SEARCH REPORT

| International application No. |
|---|
| **PCT/BR2022/050350** |

| A. CLASSIFICATION OF SUBJECT MATTER |
|---|
| **IPC: A61K31/03 (2006.01), A61K31/045 (2006.01), A61K31/047 (2006.01), A61K31/05 (2006.01), A61K8/67 (2006.01). CPC: A61K31/03; A61K31/045; A61K31/047; A61K31/05; A61K8/676; A61K2800/92.** <br> According to International Patent Classification (IPC) or to both national classification and IPC |

| B. FIELDS SEARCHED |
|---|
| Minimum documentation searched (classification system followed by classification symbols) <br><br> **A61K31/03 (2006.01), A61K31/045 (2006.01), A61K31/047 (2006.01), A61K31/05 (2006.01), A61K8/67 (2006.01).** |
| Documentation searched other than minimum documentation to the extent that such documents are included in the fields searched <br><br> **Base de patentes INPI - BR** |
| Electronic data base consulted during the international search (name of data base and, where practicable, search terms used) <br><br> **Derwent e Google Patents** |

| C. DOCUMENTS CONSIDERED TO BE RELEVANT | | |
|---|---|---|
| Category* | Citation of document, with indication, where appropriate, of the relevant passages | Relevant to claim No. |
| A | BR PI1104203 A2 (MARINS SANDRA REGINA [BR]) <br> 18 March 2014 (2014.03.18) | 1-15 |
| A | Nahas, E.P., *et al*. Benefits of soy germ isoflavones in postmenopausal women with contraindication for conventional hormone replacement therapy. Maturitas, v. 48, p. 372–380, 2004. | 1-15 |
| A | Imhof, M., *et al*. Soy germ extract alleviates menopausal hot fl ushes: placebo controlled double-blind trial. European Journal of Clinical Nutrition, v. 72, p. 961 – 970, 2018. | 1-15 |
| A | Pereira, A.C.M., *et al*. Co-Treatment of Purified Annatto Oil (Bixa orellana L.) and Its Granules (Chronic®) Improves the Blood Lipid Profile and Bone Protective Effects of Testosterone in the Orchiectomy-Induced Osteoporosis in Wistar Rats. Molecules, v. 26, p. 4720, 2021. | 1-15 |

| [X] Further documents are listed in the continuation of Box C. | [ ] See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18/11/2022** | **01/12/2022** |

| Name and mailing address of the ISA/ | Authorized officer |
|---|---|
| INSTITUTO NACIONAL DA PROPRIEDADE INDUSTRIAL <br> Rua Mayrink Veiga n° 9, 6° andar <br> Facsimile No. cep: 20090-910, Centro - Rio de Janeiro/RJ | Telephone No. |

Form PCT/ISA/210 (second sheet) (January 2015)

**INTERNATIONAL SEARCH REPORT**

International application No.

PCT/BR2022/050350

C (Continuation).    DOCUMENTS CONSIDERED TO BE RELEVANT

| Category* | Citation of document, with indication, where appropriate, of the relevant passages | Relevant to claim No. |
|---|---|---|
| A | ------------------------------------------------<br>Fidelis, M., *et al*. In vitro antioxidant and antihypertensive compounds from camu-camu (Myrciaria dubia McVaugh, Myrtaceae) seed coat: A multivariate structure - activity study. Food and Chemical Toxicology, v. 120, p. 479–490, 2018.<br>------------------------------------------------ | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ABDUL-MAJEED S. ; N. MOHAMED ; I.N. SOELAIMAN.** Effects of tocotrienol and lovastatin combination on osteoblast and osteoclast activity in estrogen-deficient osteoporosis. *Evid Based Complement Alternat Med,* 2012, vol. 2012, 960742
- **BHASIN S ; CALOF OM ; STORER TW ; LEE ML ; MAZER NA ; JASUJA R et al.** Drug insight: Testosterone and selective androgen receptor modulators as anabolic therapies for chronic illness and aging. *Nat Clin Pract Endocrinol Metab,* 2006, vol. 2 (3), 146-59
- **BONFIM MR ; OLIVEIRA ASB ; AMARAL SL ; MONTEIRO HL.** Tratamento das Dislipidemias com Estatinas e Exercícios Físicos: Evidências Recentes das Respostas Musculares. *Arq Bras Cardiol,* 2015, vol. 104 (4), 324-332
- **CARVALHO, HO ; FARIAS E SOUZA, BS ; SANTOS, IVF ; RESQUE, RL ; KEITA, H ; FERNANDES, CP ; CARVALHO, JCT.** Hypoglycemic effect of formulation containing hydroethanolic extract of Calophyllum brasiliense in diabetic rats induced by streptozotocin. *Rev bras farmacogn,* 2016
- **CHIN K.Y. S.** Ima-Nirwana, Effects of annatto-derived tocotrienol supplementation on osteoporosis induced by testosterone deficiency in rats. *Clin Interv Aging,* 2014, vol. 9, 1247-1259
- **DENG, L. ; DING, Y. ; PENG, Y. ; WU, Y. ; FAN, J. ; LI, W. ; FU, Q.** γ-Tocotrienol protects against ovariectomy-induced bone loss via mevalonate pathway as HMG-CoA reductase inhibitor. *Bone,* 2014, vol. 67, 200-207
- **DOBLARÉ, M. ; GARCI, J. M. ; GOMÉZ, M. J.** Modelling bonne tissue fracture and heling a review. *Engineering Fracture Mechanics, Oxford,* 2004, vol. 71, 1809-1840
- Ovariectomy/Orchidectomy in Rodents. **IDRIS A.I.** Bone Research Protocols. Methods in Molecular Biology (Methods and Protocols). Humana Press, 2012, vol. 816

- **MANOLAGAS SC ; KOUSTENI S ; JILKA RL.** Sex steroids and bone. *Recent Prog Horm Res,* 2002, vol. 57, 385-409
- **PALMA, M. N. N. ; ROCHA, G. C. ; VALADARES FILHO, S. C. ; DETMANN E.** Evaluation of Acid Digestion Procedures to Estimate Mineral Contents in Materials from Animal Trials. Asian Australas. *J. Anim. Sci.,* 2015, vol. 28 (11), 1624-1628
- **PEARCE BC ; PARKER RA ; DEASON ME ; QURESHI AA ; WRIGHT JJ.** Hypocholesterolemic activity of synthetic and natural tocotrienols. *J Med Chem.,* 1992, vol. 35, 3595-606
- **QURESHI AA ; KHAN DA ; SALEEM S ; SILSWAL N ; TRIAS AM ; TAN B et al.** Pharmacokinetics and Bioavailability of Annatto δ-tocotrienol. *Healthy Fed Subjects,* 2015, vol. 6
- **RIGGS BL ; KHOSLA S ; MELTON LJ 3RD.** A unitary model for involutional osteoporosis: estrogen deficiency causes both type I and type II osteoporosis in postmenopausal women and contributes to bone loss in aging men. *J Bone Miner Res.,* 1998, vol. 13 (5), 763-73
- **SAKAMOTO K ; KIMURA J.** Mechanism of statin-induced rhabdomyolysis. *J Pharmacol Sci.,* 2013, vol. 123, 289-94
- **SOUZA MO ; SILVA M ; SILVA ME ; OLIVEIRA RP ; PEDROSA ML.** Diet supplementation with acai (Euterpe oleracea Mart.) pulp improves biomarkers of oxidative stress and the serum lipid profile in rats. *Nutrition.,* 2010, vol. 26, 804-810
- **TAN B ; WATSON RR ; PREEDY VR.** Tocotrienols: Vitamin E Beyond Tocopherols. 2013
- **VANDERSCHUEREN D ; LAURENT MR ; CLAESSENS F ; GIELEN E ; LAGERQUIST MK ; VANDENPUT L et al.** Sex steroid actions in male bone. *Endocr Rev.,* 2014, vol. 35 (6), 906-60
- **WONG, S. K. ; CHIN, K.-Y. ; SUHAIMI, F. H. ; AHMAD, F. ; IMA-NIRWANA, S.** Exploring the potential of tocotrienol from Bixa orellana as a single agent targeting metabolic syndrome and bone loss. *Bone,* 2018, vol. 116, 821